(19) Europäisches Patentamt
European Patent Office
Office européen des brevets

(11) Veröffentlichungsnummer: **0 019 688**
**A1**

# (12) EUROPÄISCHE PATENTANMELDUNG

(21) Anmeldenummer: **80101323.6**

(22) Anmeldetag: **29.06.78**

(51) Int. Cl.³: **C 07 D 233/84**, C 07 D 233/42, C 07 D 401/04, C 07 D 409/04 // C07D513/04

(30) Priorität: **07.07.77 LU 77703**

(43) Veröffentlichungstag der Anmeldung: **10.12.80 Patentblatt 80/25**

(84) Benannte Vertragsstaaten: **BE CH DE FR GB NL SE**

(71) Anmelder: **CIBA-GEIGY AG, Patentabteilung Postfach, CH-4002 Basel (CH)**

(72) Erfinder: **Göschke, Richard, Dr., Felixhäglistrasse 21, CH-4103 Bottmingen (CH)**
Erfinder: **Ferrini, Pier Giorgio, Dr., Im Rehwechsel 22, CH-4102 Binningen (CH)**

(74) Vertreter: **Zumstein sen., Fritz, Dr. et al, Bräuhausstrasse 4, D-8000 München 2 (DE)**

## (54) Diazaverbindungen und Verfahren zu deren Herstellung.

(57) Die Erfindung betrifft neue Diazaverbindungen, insbesondere 1,3-Diaza-cyclopent-2-ene der allgemeinen Formel (I)

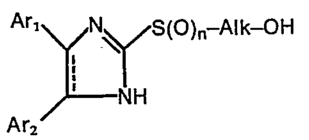

(I),

deren 1,3-Diaza-cyclopent-2-en-ring eine weitere Doppelbindung aufweisen kann, Alk Niederalkylen darstellt, welches das Thia- vom Aza-atom durch 2–4 Kohlenstoffatome trent, $Ar_1$ und $Ar_2$ unabhängig voneinander gegebenenfalls substituiertes Phenyl, Pyridyl oder Thienyl bedeuten und n 0, 1 oder 2 ist, mit der Maßgabe, daß mindestens einer der Reste $Ar_1$ und $Ar_2$ von Phenyl verschieden ist, wenn Alk Äthylen und der 1,3-Diaza-cyclopent-2-en-ring einen Imidazolring darstellt, und deren Salze, sowie Verfahren zu ihrer Herstellung.

Die neuen Verbindungen können als Zwischenprodukte von Arzneimittelwirkstoffen verwendet werden, insbesondere entzündungshemmenden und antirheumatischen Wirkstoffen.

EP 0 019 688 A1

0019688

CIBA-GEIGY AG
Basel (Schweiz)

4-11226/+/C
Europa

BEZEICHNUNG GEÄNDERT
siehe Titelseite

Neue Diazaverbindungen und Verfahren zu deren Herstellung und ihre Verwendung

Die Erfindung betrifft neue Diazaverbindungen, insbesondere 1,3-Diaza-cyclopent-2-ene der allgemeinen Formel (I)

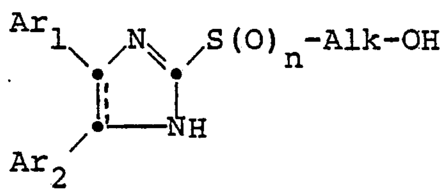

(I),

deren 1,3-Diaza-cyclopent-2-en-ring eine weitere Doppelbindung aufweisen kann, Alk Niederalkylen darstellt, welches das Thia- vom Aza-atom durch 2-4 Kohlenstoffatome trennt, $Ar_1$ und $Ar_2$ unabhängig voneinander gegebenenfalls substituiertes Phenyl, Pyridyl oder Thienyl bedeuten und n O, 1 oder 2 ist, mit der Massgabe, dass mindestens einer der Reste $Ar_1$ und $Ar_2$ von Phenyl verschieden ist, wenn Alk Aethylen und der 1,3-Diaza-cyclopent-2-en-ring einen Imidazolring darstellt, und deren Salze, sowie Verfahren zu ihrer Herstellung.

Die im Zusammenhang mit der vorliegenden Beschreibung mit "nieder" bezeichneten Reste und Verbindungen enthalten vorzugsweise bis 7 und in erster Linie bis 4 Kohlenstoffatome.

Niederalkylen Alk ist vorzugsweise unverzweigtes, aber auch verzweigtes Niederalkylen mit 2-4 Kohlenstoffatomen in der Kette zwischen dem Schwefel- und dem Stickstoffatom.

Pyridyl ist ein 2-, 3- oder 4-Pyridyl und Thienyl ein 3- oder insbesondere 2-Thienyl.

Substituiertes Phenyl, Pyridyl oder Thienyl ist z.B. einfach, zweifach oder auch mehrfach substituiert. Substituenten, insbesondere am Phenylrest sind u.a. Niederalkyl, Niederalkoxy, Halogen, Trifluormethyl, Niederalkylsulfonyl oder Nitro. Substituenten am Pyridyl- oder Thienyl-rest sind vorzugsweise Niederalkyl, Halogen oder Trifluormethyl.

Vorstehend wie nachfolgend können die Allgemeinbegriffe folgende Bedeutung haben:

Niederalkyl ist z.B. Methyl, Aethyl, n-Propyl, Isopropyl, n-Butyl, Isobutyl, sec.-Butyl oder tert.-Butyl, ferner n-Pentyl, n-Hexyl, Isohexyl oder n-Heptyl.

Niederalkylen ist Aethylen, sowie 1,3-Propylen, 1,4-Butylen, kann aber auch 1,2-Propylen, 1,2- oder 2,3-Butylen, 1,3- oder 2,4-Pentylen oder 1,4-Pentylen sein.

Niederalkoxy ist z.B. Methoxy, Aethoxy, n-Propyloxy, Isopropyloxy, n-Butyloxy oder tert.-Butyloxy.

Halogen ist solches mit der Atomnummer bis und mit 35 und steht für Fluor oder Brom, vorzugsweise für Chlor.

Niederalkylsulfonyl steht z.B. für Methylsulfonyl, Aethylsulfonyl oder n-Propylsulfonyl.

Die erfindungsgemässen Verbindungen dienen als Zwischenprodukte für die Herstellung von Verbindungen der Formel

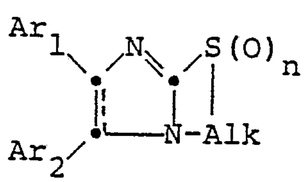

(Ia),

worin $Ar_1$, $Ar_2$, n und Alk obige Bedeutungen haben, die ihrerseits wertvolle pharmakologische Eigenschaften besitzen, insbesondere entzündungshemmende und antirheumatische Wirkungen, wie sich in Tierversuchen zeigen lässt, z.B. im Kaolin-Pfotenoedem-Test (Helv. Physiol. Acta 25 (1967) 156) an der Ratte bei einer peroral gegebenen Dosis ab etwa 10 mg/kg oder im Terpentin-Pleuritis-Test [Helv. Physiol. Acta 26 (1969) 287] an der Ratte peroral gegeben bei einer Dosis von 30 bis 100 mg/kg, ferner im Adjuvans-Arthritis-Test [Pharmacology 2 (1969) 288] an der Ratte bei einer peroralen Dosis von 10-30 mg/kg, im Phenyl-p-benzochinon-Test an der Maus (Proc. Soc. Exp. Biol. 95 (1957) 729) bei Dosen von 30 bis 100 mg/kg, peroral gegeben, und im Pertussisoedem-Test (Agents and Actions, vol. 6, 613,1976) bei 5-50 mg/kg Ratte.

Die Erfindung betrifft insbesondere Verbindungen der Formel I, worin $Ar_1$ und $Ar_2$ unabhängig voneinander einen gegebenenfalls durch Niederalkyl, Niederalkoxy, Halogen oder Trifluormethyl substituierten Phenylrest, einen Pyridyl-, wie einen 2-, 3- oder 4-Pyridyl-, oder Thienyl-, insbesondere einen 2-Thienylrest darstellen, Alk einen Niederalkylenrest, der das Schwefel- und Stickstoffatom über 2-3 Kohlenstoffatome miteinander verbindet, in erster Linie einen unverzweigten Niederalkylenrest und n besonders 0, ferner auch 1 oder 2 bedeuten, und deren Salze.

Die Erfindung betrifft in erster Linie Verbindungen der Formel II

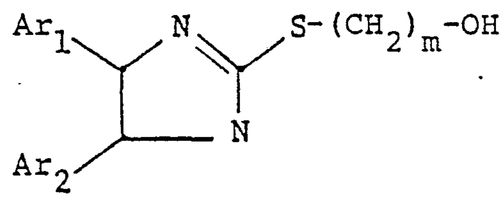

(II)

worin $Ar_1$ und $Ar_2$ unabhängig voneinander einen gegebenenfalls durch Niederalkoxy, wie Methoxy oder Halogen, insbesondere Chlor substituierten Phenylrest bedeuten und m in erster Line 2, ferner auch 3 ist, und ihre Salze.

Die Erfindung betrifft aber besonders auch Verbindungen der Formel III

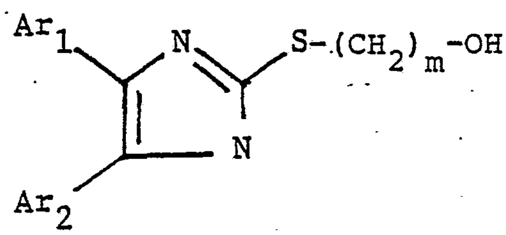

(III)

worin $Ar_1$ und $Ar_2$ unabhängig voneinander einen gegebenenfalls durch Niederalkoxy, wie Methoxy oder Halogen, insbesondere Chlor substituierten Phenylrest bedeuten und m in erster Line 2, ferner auch 3 ist, und ihre Salze.

Insbesondere betrifft die Erfindung die neuen in den Beispielen beschriebenen Verbindungen.

Die neuen Verbindungen lassen sich nach an sich bekannten Methoden herstellen z.B. indem man Verbindungen der Formeln IV und V

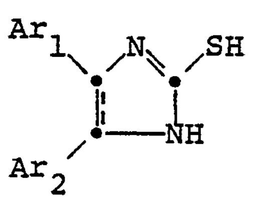

(IV) und X-Alk-OH (V)

worin X eine reaktionsfähige veresterte Hydroxygruppe darstellt, miteinander umsetzt, und, wenn erwünscht, in der erhaltenen Verbindung, worin n O ist, das Thiaatom zur Sulfinyl- oder Sulfonylgruppe oxidiert, und/oder, wenn erwünscht, eine erhaltene freie Verbindung in eines ihrer Salze überführt oder ein erhaltenes Salz in die freie Verbindung umwandelt.

Reaktionsfähig verestertes Hydroxy ist insbesondere eine mit einer starken anorganischen Säure, z.B. Halogenwasserstoff, insbesondere Chlorwasserstoff, oder Schwefelsäure, oder mit einer starken organischen Säure, wie mit einer Niederalkansulfonsäure, z.B. Methan- oder Aethansulfonsäure oder einer gegebenenfalls durch Niederalkyl, Niederalkoxy oder Halogen substituierten Benzolsulfonsäure, z.B. p-Toluolsulfonsäure oder p-Brombenzolsulfonsäure veresterte Hydroxygruppe.

Die obige Kondensation wird vorzugsweise unter säureabspaltenden Bedingungen durchgeführt.Dabei arbeitet man in erster Linie in einem niedersiedenden Lösungsmittel, wie Dimethylformamid oder Aceton, einem Alkohol, z.B. Methanol oder Aethanol, wenn erwünscht, in Gegenwart einer Base, z.B. einer anorganischen Base, wie einem Alkali- oder Erdalkalihydrid, -hydroxyd oder -carbonat, in erster Linie Natriumhydrid, Natriumhydroxyd oder Natriumcarbonat, oder einer organischen Base, vorzugsweise einer Stickstoffbase, wie Triniederalkylamin, z.B. Trimethylamin, Triäthylamin, Dimethyl-isopropylamin, oder Pyridin.

Die Oxydation des Thia-atoms zur Sulfinyl- oder Sulfonylgruppe lässt sich in an sich bekannter Weise z.B. mit Peroxyden, wie Wasserstoffperoxyd, oder Persäuren, z.B. einer gegebenenfalls durch Niederalkyl, Niederalkoxy, Halogen oder einer weiteren Carboxylgruppe substituierten Benzoepersäure, wie Benzoepersäure selbst oder Phthalmonopersäure, oder einer Alkanpercarbonsäure, wie Peressigsäure oder einem Perjodat, wie Natriumperjodat durchführen. Diese Reaktion wird meist bei tiefen Temperaturen in einem Lösungsmittel, wie Eisessig oder Aceton vorgenommen.

Die neuen Verbindungen können in Form von Säureadditionssalzen, insbesondere pharmazeutisch verwendbaren, nicht-toxischen Salzen, z.B. mit organischen Säuren, wie Chlorwasserstoff-, Bromwasserstoff-, Schwefel- oder Phosphorsäure, oder mit organischen, wie aliphatischen, cycloaliphatischen, cycloaliphatisch-aliphatischen, aromatischen, araliphatischen, heterocyclischen oder heterocyclisch-aliphatischen Carbon- oder Sulfonsäure, z.B. Essig-, Propion-, Bernstein-, Glykol-, Milch-, Aepfel-, Wein-, Zitronen-, Ascorbin-, Malein-, Phenylessig-, Benzoe-, 4-Aminobenzoe-, Anthranil-, 4-Hydroxybenzoe-, Salicyl-, Aminosalicyl-, Embon- oder Nicotin-, sowie Methansulfon-, Aethansulfon-, 2-Hydroxyäthansulfon-, Aethylensulfon-, Benzolsulfon-, p-Toluolsulfon-, Naphthalinsulfon-, Sulfanil- oder Cyclohexylsulfaminsäure, vorliegen. Salze dieser Art können z.B. durch Behandeln der freien Verbindungen, mit den Säuren oder mit geeigneten Anionenaustauscherharzen erhalten werden.

Infolge der engen Beziehungen zwischen den neuen Verbindungen in freier Form und in Form ihrer Salze sind im Vorausgegangenen und nachfolgend unter den freien Verbindungen oder den Salzen sinn- und zweckmässig gegebenenfalls auch die entsprechenden Salze bzw. freien Verbindungen zu verstehen.

Die neuen Verbindungen können als Isomerengemische, wie Racemate oder Diastereoisomerengemische, oder in Form der reinen Isomeren, wie optisch aktiven Komponenten, vorliegen. Die Auftrennung von erhaltenen Isomerengemischen in die reinen Isomeren kann nach den bekannten Methoden geschehen. Racemate lassen sich z.B. auf Grund physikalisch-chemischer Unterschide, wie z.B. solchen der Löslichkeit, ihrer diastereomeren Salze, oder

durch fraktioniertes Kristallisieren aus einem optisch aktiven Lösungsmittel, oder durch Chromatographie, insbesondere Dünnschichtchromatographie, an einem optisch aktiven Trägermaterial, in die optisch aktiven Antipoden auftrennen. Dabei isoliert man vorteilhafterweise das pharmakologisch wirksamere oder weniger toxische reine Isomere, insbesondere den wirksameren oder weniger toxischen aktiven Antipoden.

Die obigen Reaktionen werden in üblicher Weise in An- oder Abwesenheit von Verdünnungs-, Kondensations- und/oder katalytischen Mitteln, falls notwendig, bei erniedrigter oder erhöhter Temperatur, im geschlossenen Gefäss und/oder in einer Inertgasatmosphäre durchgeführt.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den eingangs als besonders bevorzugt aufgeführten Verbindungen gelangt.

Die Verbindungen der Formel I können, wie erwähnt, zur Herstellung pharmakologisch wirksamer Verbindungen der Formel Ia verwendet werden, indem man diese unter Ueberführung der Hydroxygruppe in reaktionsfähig verestertes Hydroxy reaktionsfähig verestert. Diese cyclisieren unter analogen Bedingungen, wie für die Herstellung der Verbindungen der Formel I beschrieben, zu den Endprodukten der Formel Ia.

Die folgenden Beispiele dienen zur Illustration der Erfindung; Temperaturen werden in Celsiusgraden angegeben.

Beispiel 1: 2,5 g Natrium werden in 220 ml Aethanol gelöst. Dazu gibt man 35g 4,5-Dianisyl-imidazolin-2-thion. Man erhält eine Suspension. Innerhalb 2 Minuten werden dazu bei Raumtemperatur (Rühren) 15,7 ml 2-Chloräthanol getropft. Man rührt 1 Stunde bei 60°C und 4 Stunden am Rückfluss weiter. Anschliessend wird die schwache Suspension zur Trockne eingedampft. Der Rückstand wird durch Umkristallisation aus Aceton/Wasser gereinigt. Man erhält das 4,5-Di-(p-Methoxyphenyl)-2-β-hydroxyäthylmercapto-imidazol.

Beispiel 2: 0,8 g Natrium werden in 200 ml Aethanol gelöst. Die Lösung wird mit 8 g 4-Phenyl-5-(3-pyridyl)-2-mercapto-imidazol versetzt und zum Rückfluss erhitzt. Nachdem eine klare Lösung entstanden ist, werden 2,3 ml 2-Chloräthanol zugetropft und das Reaktionsgemisch wird 16 Stunden am Rückfluss gekocht. Das Lösungsmittel wird am Vakuum abgedampft und der Rückstand zwischen Wasser und Essigester verteilt. Die organischen Phasen werden mit Wasser und Sole gewaschen, mit Natriumsulfat getrocknet und eingedampft. Als Rückstand erhält man kristallines 2-(2-Hydroxyäthylthio)-4(5)-(3-pyridyl)-5(4)-phenyl-imidazol. Es schmilzt nach dem Umkristallisieren aus Essigester/Aethanol bei 157-159°C.

Das Ausgangsmaterial lässt sich wie folgt erhalten:

10,8 g Benzyl-(3-pyridyl)-keton werden zusammen mit 40 ml Pyridin und einer Lösung von 8 g Hydroxylaminhydrochlorid in 15 ml Pyridin während 6 Stunden bei 100° gerührt. Das Reaktionsgemisch wird auf Eis/Wasser gegossen

und 15 Minuten weitergerührt. Die ausgefallenen Kristalle werden abgenutscht, mit Wasser gewaschen und am Hochvakuum getrocknet. Man erhält das Benzyl-(3-pyridyl)-keton-oxim vom F. 122-126°.

Zu einer bei -10° gerührten Lösung von 8,5 g Benzyl-(3-pyridyl)-keton-oxim in 20 ml Pyridin wird innert 5 Minuten die Lösung von 7,7 g p-Toluolsulfochlorid in 15 ml Pyridin zugetropft. Das Reaktionsgemisch wird 24 Stunden im Eisschrank aufbewahrt und dann auf Eis/Wasser gegossen. Nach längerem Rühren und Verreiben erstarrt das ausgefallene Oel zu Kristallen. Diese werden abgenutscht, mit Wasser gewaschen und am Hochvakuum getrocknet. Man erhält 11,6 g eines Rohproduktes vom F. 87-92°, welches nach Dünnschichtchromatographie jedoch noch Edukt enthält. Es wird direkt in der nächsten Stufe eingesetzt.

11,6 g roher Benzyl-(3-pyridyl)-keton-oxim-p-toluolsulfoester werden in 90 ml absolutem Aethanol suspendiert und bei 0° unter Rühren die Lösung von 3,7 g Kalium-tert.-butylat in 30 ml absolutem Aethanol zugetropft. Das Reaktionsgemisch wird 2 Stunden bei 0° gerührt. Die Suspension wird abgenutscht und das Filtrat sofort in der nächsten Stufe eingesetzt.

3,6 g Natriumthiocyanat werden in 60 ml Aethanol gelöst und mit 4,5 ml konzentrierter Salzsäure versetzt. Die Suspension wird abgenutscht und das Filtrat wird zusammen mit der alkoholischen Lösung des erhaltenen α-Aminobenzyl-(3-pyridyl)-keton während 18 Stunden am Rückfluss gehalten. Nach dem Abkühlen lässt sich aus dem Reaktionsgemisch 2,8 g rohes 4-Phenyl-5-(3-pyridyl)-2-mercapto-imidazol abnutschen. Das Filtrat enthält weitere Mengen des Produktes. Nach dem Umkristallisieren aus Dimethylformamid-Wasser schmilzt es bei 290-300°.

In analoger Weise, ausgehend von den entsprechenden Ausgangsstoffen lassen sich die folgenden Verbindungen herstellen:

das 4,5-Di-(p-methylphenyl)-2-(2-hydroxyäthylthio)-4-imidazolin,

das 4,5-Di-(p-chlorphenyl)-2-(2-hydroxyäthylthio)-4-imidazolin,

das 4,5-Di-(m-chlorphenyl)-2-(2-hydroxyäthylthio)-4-imidazolin,

das 4,5-Di-(p-methoxyphenyl)-2-(2-hydroxypropyl)-4-imidazolin und

das 4,5-Di-(p-methoxyphenyl)-2-(3-hydroxypropyl)-4-imidazolin.

Beispiel 3: 0,69 g Natrium werden in 60 ml Aethanol gelöst und mit 9,36 g 2-Mercapto-4,5-di-p-methoxyphenyl-imidazol versetzt. Das Reaktionsgemisch wird 30 Minuten gerührt, mit 3,1 g 3-Chlor-1-propanol versetzt, 2 Stunden am Rückfluss gehalten, abgekühlt, klar-filtriert und zur Trockne eingedampft. Als Rückstand bleibt das rohe 2-(3-Hydroxypropylthio)-4,5-di-(p-methoxyphenyl)-imidazol zurück.

Beispiel 4: Analog Beispiel 3 wird aus dem 2-Mercapto-4,5-di-p-chlorphenyl-imidazol mit 2-Chloräthanol das 2-(2-Hydroxyäthylthio )-4,5-di-p-chlorphenyl-imidazol vom F. 197-199° hergestellt, das analog Beispiel 7 durch Cyclisation mit Thionylchlorid zu 5,6-Di-p-chlorphenyl-2,3-dihydro-imidazol[2,1-b]-thiazol vom F.199-204° verarbeitet werden kann.

Beispiel 5: 14,8 g 4,5-Di-(p-Methoxyphenyl)-2-β-hydroxyäthylmercapto-imidazol werden in 50 ml absolutem Pyridin gelöst und unter Rühren bei ca. -5° Innentemperatur zu

einer Lösung von 15,8 g Benzolsulfochlorid in 60 ml absolutem Pyridin getropft. Anschliessend rührt man 60 Stunden bei 0° aus. Das Reaktionsgemisch wird auf Eiswasser gegossen und mit Dichlormethan extrahiert. Die mit Wasser 3-mal gewaschenen und vereinigten Dichlormethanextrakte werden über Natriumsulfat getrocknet und zur Trockne eingedampft. Das viskose Oel wird aus Toluol-Petroläther kristallisiert. Das so erhaltene 5,6-Di-(p-methoxyphenyl)-2,3-dihydro-imidazo[2,1-b]thiazol vom F. 152-154°.

Beispiel 6: Zu einer Lösung von 23 g p-Toluolsulfochlorid in 80 ml Pyridin wird bei 0° unter Rühren die Lösung von 20 g 2-(2-Hydroxyäthylthio)-4(5)-(3-pyridyl)-5(4)-phenylimidazol zugegeben. Das Reaktionsgemisch wird 16 Stunden bei 0° gerüht, auf Eis-Wasser gegossen und mit Aether extrahiert. Aus den Aetherextrakten erhält man nach dem Waschen mit Wasser und Trocknen über Natriumsulfat einen Eindampfrückstand der nach dem Chromatographieren auf Silicagel mit Toluol und Essigester und nach dem Umkristallisieren der entsprechenden Fraktionen aus Essigester das Isomerengemisch 5(6)-(3-Pyridyl)-6(5)-phenyl-2,3-dihydro-imidazo[2,1-b]thiazol als weisse Kristalle vom F. 150-151° ergibt.

Beispiel 7: Das gemäss Beispiel 3 erhältliche rohe 2-(3-Hydroxypropyl-4,5-di-(p-methoxyphenyl)-imidazol wird in 50 ml Thionylchlorid 30 Minuten am Rückfluss gehalten. Das überschüssige Thionylchlorid wird abgedampft, durch Abdestillieren von 100 ml noch zugesetztem Chloroform abgetrieben und der Rückstand zusammen mit 100 ml Aethanol und 50 ml 40%-iger Kalilauge 4 Stunden am Rückfluss gehalten. Danach wird das Reaktionsgemisch am Rotationsverdampfer eingedampft, mit Eiswasser versetzt und mit Essigester

extrahiert. Die organischen Phasen werden mit Wasser gewaschen, getrocknet und eingedampft. Der Rückstand wird mit Toluol: Essigester 1:1 auf Silicagel chromatographiert. Durch Umkristallisieren der entsprechenden Fraktionen aus Aceton erhält man das 6,7-Di-p-methoxyphenyl-2,3,4,5-tetrahydro-imidazo[2,1-b](1,3)-thiazin vom F. 189-191°.

Patentansprüche:

1. 1,3-Diaza-cyclopent-2-ene der allgemeinen Formel I

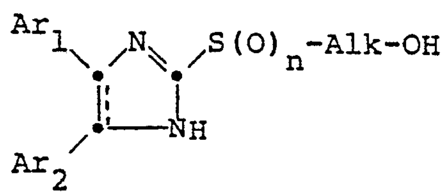

(I),

deren 1,3-Diaza-cyclopent-2-en-ring eine weitere Doppelbindung aufweisen kann, Alk Niederalkylen darstellt, welches das Thia- vom Aza-atom durch 2-4 Kohlenstoffatome trennt, $Ar_1$ und $Ar_2$ unabhängig voneinander gegebenenfalls substituiertes Phenyl, Pyridyl oder Thienyl bedeuten und n 0, 1 oder 2 ist, mit der Massgabe, dass mindestens einer der Reste $Ar_1$ und $Ar_2$ von Phenyl verschieden ist, wenn Alk Aethylen und der 1,3-Diaza-cyclopent-2-en-ring einen Imidazolring darstellt, und deren Salze.

2. Verbindungen gemäss Anspruch 1 der Formel I, worin $Ar_1$ und $Ar_2$ unabhängig voneinander einen gegebenenfalls durch Niederalkyl, Niederalkoxy, Halogen oder Trifluormethyl substituierter Phenylrest, einen Pyridyl-oder Thienylrest darstellen, Alk ein Niederalkylenrest, der das Schwefel- und Stickstoffatom über 2-3 Kohlenstoffatome miteinander verbindet,und n 0, 1 oder 2 bedeutet und ihre Salze.

3. Verbindungen gemäss Anspruch 1 der Formel II

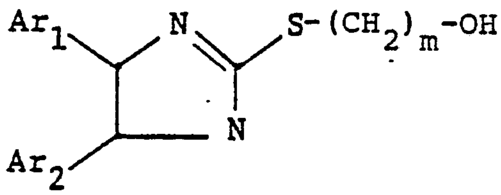

(II)

worin $Ar_1$ und $Ar_2$ unabhängig voneinander einen gegebenenfalls durch Niederalkoxy, wie Methoxy oder Halogen, insbesondere Chlor, substituierten Phenylrest bedeuten und m in erster Linie 2, ferner auch 3 ist, und ihre Salze.

4. 2-(2-Hydroxyäthylthio)-4,5-di-(p-methyl-phenyl)-imidazolin und dessen Salze.

5. 2-(2-Hydroxyäthylthio)-4,5-di-(p-chlor-phenyl)-imidazolin und dessen Salze.

6. 2-(2-Hydroxyäthylthio)-4,5-di-(m-chlor-phenyl)-imidazolin und dessen Salze.

7. 2-(2-Hydroxypropylthio)-4,5-di-(p-methoxy-phenyl)-imidazolin und dessen Salze.

8. 2-(3-Hydroxypropylthio)-4,5-di-(p-methoxy-phenyl)-imidazolin und dessen Salze.

9. Verbindungen gemäss Anspruch 1 der Formel III

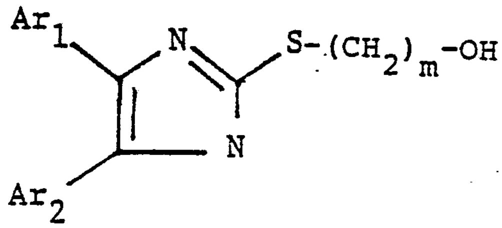

(III)

worin $Ar_1$ und $Ar_2$ unabhängig voneinander einen gegebenenfalls durch Niederalkoxy, wie Methoxy oder Halogen, insbesondere Chlor, substituierten Phenylrest bedeuten und m in erster Linie 2, ferner auch 3 ist, und ihre Salze.

10. 2-(2-Hydroxyäthylthio)-4,5-di-(p-methoxyphenyl)-imidazol und dessen Salze.

11. 2-(3-Hydroxypropylthio)-4,5-di-(p-methoxyphenyl)-imidazol und dessen Salze.

12. 4,5-Di-(p-chlorphenyl)-2-(2-hydroxyäthylthio)-imidazol und dessen Salze.

13. 2-(2-Hydroxyäthylthio)-4(5)-(3-p-pyridyl)-5(4)-phenyl-imidazol und dessen Salze.

14. Verfahren zur Herstellung von 1,3-Diaza-cyclopent-2-enen der allgemeinen Formel I

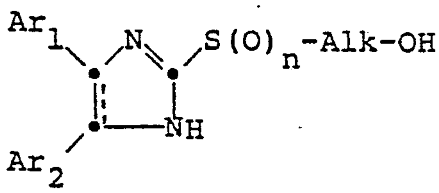

(I),

deren 1,3-Diaza-cyclopent-2-en-ring eine weitere Doppelbindung aufweisen kann, Alk Niederalkylen darstellt, welches das Thia- vom Aza-atom durch 2-4 Kohlenstoffatome trennt, $Ar_1$ und $Ar_2$ unabhängig voneinander gegebenenfalls substituiertes Phenyl, Pyridyl oder Thienyl bedeuten und n 0, 1 oder 2 ist, mit der Massgabe, dass mindestens einer der Reste $Ar_1$ und $Ar_2$ von Phenyl verschieden ist, wenn Alk

Aethylen und der 1,3-Diaza-cyclopent-2-en-ring einen Imidazolring darstellt, und deren Salze, dadurch gekennzeichnet, dass man Verbindungen der Formeln IV und V

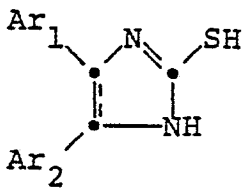 (IV) und X-Alk-OH (V)

worin X eine reaktionsfähige veresterte Hydroxygruppe darstellt, miteinander umsetzt, und, wenn erwünscht, in den erhaltenen Verbindungen der Formel I, worin n 0 ist, das Thiaatom zur Sulfinyl- oder Sulfonylgruppe oxidiert, und/oder, wenn erwünscht, eine erhaltene freie Verbindung in eines ihrer Salze überführt oder ein erhaltenes Salz in die freie Verbindung umwandelt.

Europäisches Patentamt

# EUROPÄISCHER RECHERCHENBERICHT

0019688

Nummer der Anmeldung
EP 80 10 1323

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | DE - A - 2 635 876 (DU PONT)<br>* Seiten 3 bis 8 *<br>-- | 1 |
| A | DE - A - 2 402 672 (SEARLE)<br>* Seiten 1 bis 11 *<br>-- | 1 |
| A | US - A - 2 981 739 (BIMBER)<br>* Spalte 1 bis Spalte 3 *<br>-- | 1 |
| A | US - A - 3 274 209 (RAEYMAEKERS et al.)<br>* Spalte 1 bis Spalte 4 *<br>-- | 1,14 |
| A | US - A - 3 364 112 (RAEYMAKERS et al.)<br>* Spalte 1 bis Spalte 4 *<br>---- | 1,14 |

KLASSIFIKATION DER ANMELDUNG (Int.Cl. 3)

C 07 D 233/84
233/42
401/04
409/04//
513/04

RECHERCHIERTE SACHGEBIETE (Int. Cl. 3)

C 07 D 233/84
233/42
401/04
409/04

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 03-07-1980 | DE BUYSER |